# EUROPEAN PATENT SPECIFICATION

(11) **EP 3 305 354 B1**
(45) Date of publication and mention of the grant of the patent: **02.12.2020**
(21) Application number: 16306330.8
(22) Date of filing: 07.10.2016
(51) Int. Cl.: A61M 16/06

(54) **HOLLOW CURVED CONNECTOR WITH SEVERAL ABUTMENT STRUCTURES FOR A RESPIRATORY MASK**
HOHLER GEKRÜMMTER VERBINDER MIT MEHREREN SEKUNDÄRTEILSTRUKTUREN FÜR EINE ATEMMASKE
CONNECTEUR INCURVÉ CREUX POURVU DE PLUSIEURS STRUCTURES DE BUTÉE POUR UN MASQUE RESPIRATOIRE

(43) Date of publication of application: 11.04.2018
(73) Proprietor: Air Liquide Medical Systems, 92160 Antony (FR)
(72) Inventor: ALBERICI, Luca, 25030 Roncadelle (BS) (IT); MASSERDOTTI, Fulvio, 25075 Brescia (IT); FRANZONI, Mattia, 25021 Bagnolo Mella (Bs) (IT); BUGATTI, Ottorino, 25068 Sarezzo (Bs) (IT)
(74) Representative: Pittis, Olivier

(56) References cited:
- EP-A1- 2 708 258
- EP-A1- 2 954 919
- EP-A1- 2 954 920
- WO-A1-2010/135785
- WO-A1-2016/124145

## Description

The invention concerns an improved connecting system or mechanism for connecting together the hollow gas connector, the front piece, i.e. shroud or retainer, and the mask body of a respiratory mask, in particular a facial mask covering the nose and mouth of a patient, in particular for adult or pediatric uses in the treatment of respiratory conditions or diseases affecting adults, children, toddlers, infants or newborns.

Respiratory masks are commonly used for delivering non-invasive positive pressure ventilation (NPPV) or for continuous positive airway pressure (N-CPAP) therapy in disordered breathing conditions, such as obstructive sleep apnea (OSA), chronic obstructive pulmonary disease (COPD).

In both cases, the mask delivers a flow of breathable gas for or to assist in patient respiration. Examples of masks are given by EP-A-462701, EP-A-462701, EP-A-874667, EP-A-1972357 and WO-A-00/57942.

Actually, a mask typically comprises a rigid or semi-rigid hollow shell, usually made of polymer or silicone, defining a breathing chamber that receives at least a part of the patient's nose in case of a nasal mask, or both the nose and the mouth of the patient in case of a facial mask. The hollow shell or body receives the gas from a gas supply line, fixed to an inlet orifice arranged in the mask body by means of a hollow gas connector, for delivering the respiratory gas, such as air under pressure, into the breathing chamber of the shell.

A soft face-contacting cushion comes into contact with the patient's face and conforms to the various facial contours of the patient face thereby ensuring gas tightness. The cushion is usually made of soft, resilient elastomeric material, such as soft silicone or similar.

The mask may also include a forehead support and further a headgear for correctly positioning, maintaining and/or securing the mask on the head of a patient. The forehead support usually comprises an upwardly-projecting upper arm, that is either directly fixed to the mask body or to a supplementary front piece, usually called "retainer" or "shroud", that is arranged in front of the mask body, as disclosed by WO-A-0074758, EP-A-1057494 or EP-A-2708258.

An existing problem with masks comprising a front "retainer" or "shroud" that receives the straps of the headgear, is that the presence of that supplementary front piece increases the complexity of the mask structure and, due to this higher complexity, it has been noticed, in practice, that some patients are unable to correctly (re)assemble their mask, once that have to do it, for instance when the patients have to replace some parts of the mask that are worn out, such as the flexible cushion or any another part.

However, a wrongly assembled mask is less efficient for delivering the gas therapy and further often creates a discomfort for the patient, especially when the patient has to wear the mask overnight, as gas leaks may appear.

In other words, the problem to be solved is to provide a mask architecture comprising improved sub-part connecting system allowing an easy assembling of the sub-parts or elements constituting the mask, when they have been disassembled, i.e. uncoupled, and have to be reassembled, i.e. re-coupled, by a person, in particular the patient him/herself, thereby avoiding or at least limiting the risks of wrong mounting, and all related bad consequences for the patient, such as gas leaks, discomfort etc.

The solution of the present invention concerns a respiratory mask as defined in the claims. A respiratory mask is described comprising:
- a mask body comprising an inner chamber and a gas inlet in fluid communication with said inner chamber,
- a hollow curved connector comprising an upstream portion and a downstream portion separated by (i.e. linked by) an elbow portion (i.e. a curved portion), said hollow connector being traversed by an inner passage for a gas comprising an inlet orifice in the upstream portion and an outlet orifice in the downstream portion, said downstream portion being fluidly connected to the mask body for feeding gas into the inner chamber, and
- a front piece, also called shroud or retainer, comprising a central aperture and headgear connecting structures,
characterized in that:
- the gas inlet of the mask body and the central aperture of the front piece are traversed by the downstream portion of the hollow connector, and
- the peripheral outer wall of the downstream portion of the hollow connector is shaped, i.e. configured, so as to present a first abutment structure and a second abutment structure, said first and second abutment structures cooperating with the front piece and the mask body for holding the hollow connector and the front piece integral with the mask body, when the downstream portion of the hollow connector is inserted and retained, i.e. hold tight, into the gas inlet of the mask body.

In other words, the connecting system or mechanism for connecting together the hollow gas connector, the front piece (i.e. shroud or retainer), and the mask body of the respiratory mask according to the present invention does not require any positive connection of the front piece with the mask body. Indeed, the tight holding of those elements is ensured by the hollow curved connector that, once inserted through the aperture of the front piece and the gas inlet of the mask body, firmly maintains those elements in place, in "sandwiching" the front piece between the mask body and the first abutment structure arranged on the outer surface of the hollow curved connector, the hollow curved connector being itself hold tight in position into the gas inlet by the second abutment structure, also arranged on its outer surface, that abuts against the internal wall, i.e. the inner surface, of the mask body in the region located around the gas inlet inside the mask body.

The mask can further comprise one or more of the following additional features:
- the second abutment structure cooperates with the inner wall of the mask body in a region situated around the gas inlet, for retaining, i.e. holding, the hollow connector and the front piece integral with the mask body and, at the same time, for squeezing the front piece between the mask body and the first abutment structure.
- the first abutment structure and the second abutment structure are arranged on the peripheral outer wall of the downstream portion of the hollow connector.
- the second abutment structure abuts against the inner wall, i.e. internal surface, of the mask body so that the hollow connector is retained/hold integral with the mask body.
- the first abutment structure is sized so as to present a first gripping surface that is sufficient for abutting against the front shroud and for holding it.
- the second abutment structure is sized so as to present a second gripping surface that is sufficient for abutting against the inner wall of the mask body thereby holding the hollow curved connector integral with the mask body, while sandwiching the front shroud between the mask body and the first abutment structure.
- the second abutment structure abuts against the internal surface of the mask body in the region of the internal surface surrounding the gas inlet, i.e., on the inner side of the mask body.
- the outer wall of the mask body comprises a collar element arranged around the gas inlet of the mask body, the front piece being squeezed against the first abutment structure by said collar element. In other words, the collar element blocks and maintains the front piece in contact with the first abutment structure thereby sandwiching and squeezing said front piece against the first abutment structure, when the downstream portion of the hollow connector is inserted into the gas inlet of the mask body, while passing through said collar element, and retained therein by cooperation of the second abutment structure with the inner wall of the mask body.
- the collar element is part of and/or integral with the outer wall of the mask body.
- the collar element is made in one piece with the outer wall of the mask body, e.g., molded in one piece.
- the collar element has a tubular shape, and preferably a circular cross-section.
- the collar element is traversed by the downstream portion of the hollow connector.
- the collar element projects away from the outer surface of the mask body.
- the collar element comprises a collar inner passage.
- the first abutment structure and the second abutment structure project away from the peripheral outer wall of the downstream portion, i.e. they project away from the peripheral surface of the downstream portion of the hollow connector.
- the upstream portion and the downstream portion are linear portions separated by the elbow portion.
- the first abutment structure and the second abutment structure are arranged at a distance of at least 4 mm and/or of less than or equal to about 25 mm.
- the first abutment structure and/or the second abutment structure comprise a ring structure or at least a portion of a ring structure arranged around the peripheral surface of the downstream portion. In other words, the first abutment structure and/or the second abutment structure can have the shape of a ring or similar that entirely surrounds the peripheral outer surface of the downstream portion, or, in another embodiment, can be made of part of a ring, such as one or several segments of a ring, e.g. have a crenel-like structure.
- the downstream portion of the hollow connector is snap-fitted into the gas inlet of the mask body.
- the downstream portion of the hollow connector comprises a tubular body traversed by the inner passage and a tubular sleeve arranged around the tubular body, said tubular sleeve comprising a free open end and a blind end, and delimiting a venting passage between the tubular sleeve and the tubular body, the free open end of the tubular sleeve being arranged around the outlet orifice of the tubular body, said tubular sleeve being fixed, by the blind end, to the peripheral outer wall of the tubular body, the first abutment structure comprising said blind end of the tubular sleeve.
- the first abutment structure comprises an annular shoulder integral with the peripheral outer wall of the tubular body and projecting away from said peripheral outer wall, said annular shoulder further comprising the blind end of the tubular sleeve and a plurality of venting holes putting the venting passage in fluid communication with the atmosphere.
- the second abutment structure is arranged on the outer surface of the tubular sleeve surrounding the tubular body of the hollow connector.
- according to another embodiment, the second abutment structure is arranged directly on the outer surface of the tubular body of the hollow connector, i.e. on its peripheral surface.
- it further comprises a cushion fixed to the mask body.
- it further comprises a headgear fixed to the headgear connecting structures of the front element.
- it is a facial mask.
- the curved hollow connector is designed and adapted for conveying a gas, such as air under pressure (i.e. > 1 bar).
- the venting ports are orifices that allow venting of CO₂-enriched gases to the ambient atmosphere, i.e. gas expired the patient wearing the mask.
- at least part of the venting ports have a generally tronconical shape.
- the tubular sleeve is a tube element coaxially arranged around the tubular body of the connector.
- the plurality of venting holes are arranged in arc of circle.
- alternatively, the venting ports are distributed all along the circumference, i.e. arranged in full circle.
- the plurality of venting holes comprises at least 5 holes, preferably at least 10 holes.
- the holes having a size, such as an outlet diameter, of between about 0,3 mm and 3 mm, i.e. measured at their exit on the side of the surrounding atmosphere.
- the hollow connector has a generally curved shape, i.e. an elbow shape or an "L" shape.
- the tubular body has a generally curved shape comprising an elbow portion and linear portions sandwiching the elbow portion, namely upstream and downstream linear portions.
- the hollow connector comprising the tubular sleeve and the tubular body are molded in one piece.
- alternatively, the tubular sleeve and the tubular body are made of several components firmly fixed together, for example screwed, snap-fitted and/or glued together.
- the tubular sleeve and the tubular body are preferably made of a plastic material or similar, such as polycarbonate (PC), polypropylene (PP) or nylon.
- the hollow connector has an inner diameter of the inner gas passage of between 10 mm and 30 mm, measured at the outlet orifice.
- it further comprises an anti-asphyxia system (AAS) comprising a venting port cooperating with a mobile flap. AAS are known from US-A-5438981 and WO-A-00/38772. They are useful in facial masks in case of failure of the gas delivery apparatus.
- the mask body is a hollow body, also called "shell", comprising an inner chamber (i.e. inner compartment) and a gas inlet orifice in fluid communication with said inner chamber for allowing gas entering into said inner chamber by said inlet orifice.
- the gas inlet orifice of the mask hollow body has a diameter of between about 1 and 3 cm.
- the gas inlet orifice is arranged substantially centrally in the mask body.
- when the hollow connector according to the present invention is fluidly connected to the mask hollow body, the inner chamber of the mask body is in fluid communication with the ambient atmosphere through the venting passage and the venting ports of the hollow tubular connector.
- the tubular connector comprises a tubular upstream portion for receiving and securing thereto a hose or gas line by means of a connecting piece, such as intermediary connector that is rotatable with respect to the elbow-shape tubular connector.
- when the tubular hollow connector is inserted into the gas inlet orifice of the mask body, the gas passage traversing the hollow connector is in fluid communication with the inner chamber of the mask body for delivering respiratory gas therein, whereas said inner chamber of the mask body is further in fluid communication with the venting passage and with the venting holes of the hollow connector for venting expired-gases to the ambient atmosphere, i.e. out of the mask's inner chamber.
- it further comprises a flexible cushion fixed to the mask body, for instance the cushion can be snap-fitted to the mask body, or fixed thereto by means other means, such as glued to it or even molded in one-piece.
- the cushion can be either a nasal cushion receiving only the nose of the patient (i.e. in the case of a nasal mask), or a facial cushion receiving both the nose and the mouth of the patient (i.e. in the case of a facial mask).
- the mask is preferably a facial mask covering, in use, the nose and the mouth of the patient.
- preferably, the cushion is a facial cushion configured and sized for coming into contact, when the mask is worn by a user, i.e. a patient, with specific regions of the user's face, comprising a nasal bridge region including lateral nose regions, a chin region, i.e. the area located under the lower lip, and cheek regions located on each lateral sizes of the nose and mouth, said cheek regions linking the nasal bridge region including lateral nose regions to the chin region. Actually, the nasal bridge region can be an area of the nose located either at the junction of bone and cartilage, or below or above said bone/cartilage junction of the nose; the chin region is expending from the lower lip to the chin, whereas the cheek regions are the areas of the face located on right and left sizes of the nose and mouth.
- the cushion comprises a cushion body delimiting a respiratory chamber comprising a front aperture (i.e. front opening) adapted for receiving at least part of the patient's nose and/or mouth, in use, i.e., when the patient wears the mask, especially a facial mask receiving both the patient's nose and mouth.
- the cushion comprises a cushion body delimiting a respiratory chamber further comprising a rear aperture (i.e. rear opening) that opens into the inner chamber of the mask body, when the cushion is firmly fixed to the mask body, so that the respiratory chamber of the cushion is in fluid communication with the inner chamber of the mask body.
- the cushion has a generally tridimensional (3D) shape, preferably a 3D saddle, triangular or trapezoidal shape.
- the front aperture (i.e. front opening) of the cushion is configured and sized for receiving at least part of the patient's nose and/or mouth, in use.
- the cushion comprises at least one membrane forming a kind of flexible skirt along the cushion aperture receiving the patient's face, i.e. nose and/or mouth.
- the cushion comprises a membrane forming a flexible skirt along the cushion aperture that deforms so as to match the contours of the face of the patient thereby ensuring a gas tightness when the patient inserts his/her face, i.e. nose or nose and mouth, into the front aperture of said cushion.
- in another embodiment, the flexible cushion can comprise several membranes, e.g. two or three superimposed membranes that ensure the gas tightness, the outer membrane being in contact with the patient's face, when the patient wears the mask.
- the one or several membranes are arranged around all along the periphery of the front aperture of the respiratory chamber of the cushion.
- the cushion is made of a soft flexible material, preferably silicone or similar.
- the headgear connecting structures of the front shroud comprise hooks, slots, snap-fit connectors or similar, including combination thereof.
- the front shroud comprises a holding or upper arm projecting upwardly and/or two lateral arms projecting laterally. The holding arm projects upwardly, i.e. in use, in front of the nose and of part of the forehead of the patient, and/or two lateral arms projecting laterally, i.e. in use, along part of the cheeks of the patient.
- preferably, the two lateral arms projecting laterally and/or the holding arm projecting upwardly are integrally fixed to the front shroud, for instance molded in one-piece or glued together.
- the front shroud is made of a flexible material, such as a polymer material, e.g. polycarbonate (PC), polypropylene (PP) or nylon, in particular the holding arm and/or of the two lateral arms so as to be slightly bendable toward the patient's face under the forces applied by the headgear, when said headgear is adjusted to the morphology of the patient's head.
- a headgear is fixed to the headgear connecting structures.
- the two lateral arms and/or the holding arm each comprises a free distal end carrying the headgear connecting structures.
- a headgear comprising several straps is fixed to the headgear connecting structures of the two lateral arms and/or of the holding arm, preferably the straps are made of fabric or polymer materials or the like.
- the holding arm and/or of the two lateral arms have an elongated shape, for example a band or ribbon shape. Of course, other shapes are possible.
- the holding arm has a length of about 2 and 8 cm.
- each lateral arms has a length of about 3 and 6 cm.
- at least a region of the holding arm and/or of the two lateral arms is configured or shaped so as to spouse, i.e. to match, the external profile, i.e. the outer contours, of the mask body, when the front shroud is positioned in contact to the mask body. Preferably, they have a curved-shape.
- the hollow connector, preferably a curved connector, is fixed to the body mask so as to be in fluid communication with one another thereby allowing gas flows to circulate, in normal use:
   - from the hollow connector to the inner chamber of the mask body during the inspiration phases of the patient, when air under pressure is delivered to the patient,
   - and vice versa, i.e. from the mask body to the hollow connector during expiration phases, when the patient exhales CO₂-enriched gases that have to be vented to the atmosphere through the venting passage and venting holes of the tubular sleeve arranged around the tubular body of the hollow connector.
- the hollow connector is fixed to and rotatable with respect to the mask body.

The invention also concerns an assembly for delivering a gas to a patient comprising a gas delivery device, such as a medical ventilator, and a respiratory mask according to the present invention.

Preferably the gas delivery device is fluidly connected to the hollow connector and the respiratory mask by means of a gas line, such as a flexible hose, said gas line being fluidly connected to the hollow connector by means of an intermediary connector that is rotatable with respect to the hollow curved connector.

An embodiment of a facial respiratory mask according to the present invention is shown in the enclosed illustrative, but not limitative, Figures, among which:
- Figure 1 represents a general view of an embodiment of a facial mask according to the present invention,
- Figures 2 and 3 are exploded views of the facial mask of Figure 1, and
- Figures 4-8 represent different views of the connecting system according to the present invention equipping the mask of Figures 1-3.

The present invention proposes a new connecting system or mechanism for easily assembling and connecting together a hollow gas connector 1, preferably a curved connector, a shroud 24 and a mask body 21 of a respiratory mask as shown in the enclosed Figures that illustrate one embodiment of a facial mask 20 according to the present invention.

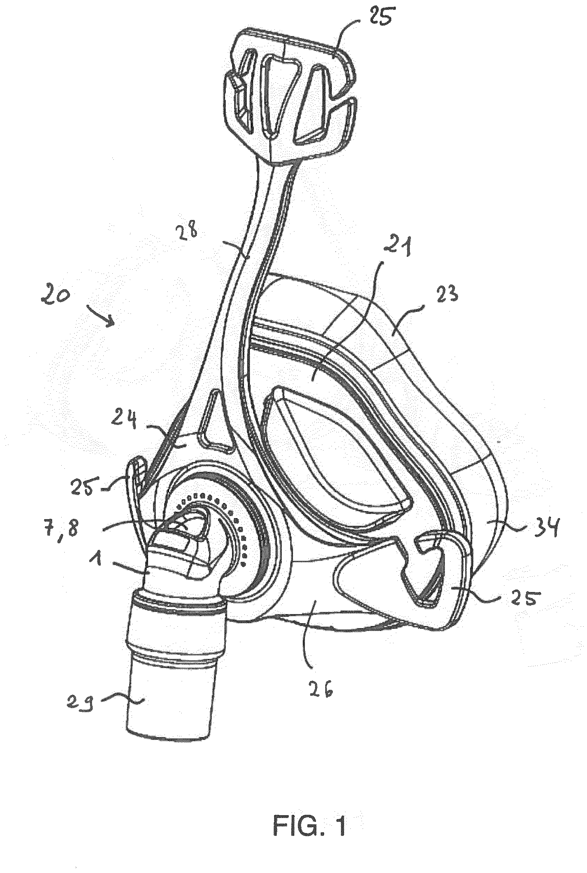

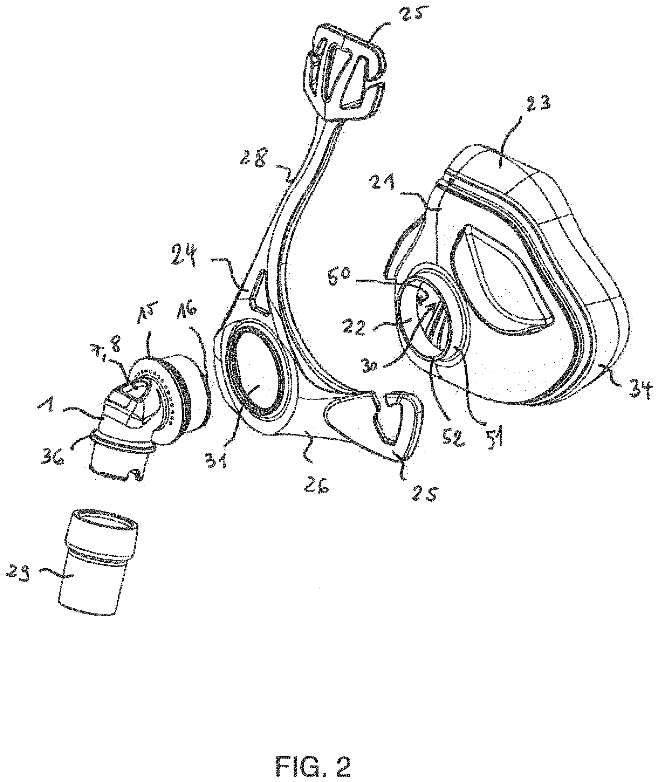

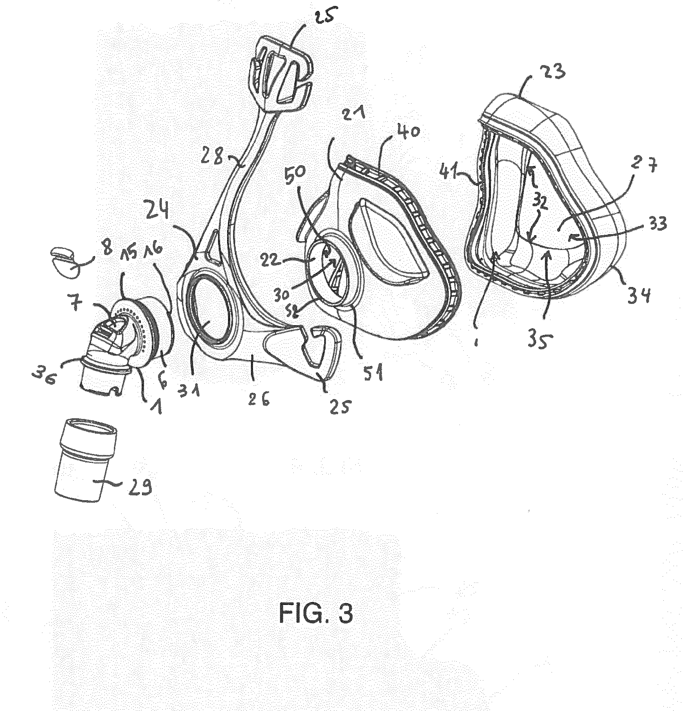

Figures 1 to 3 represent a general view of an embodiment of a respiratory mask 20, namely a facial mask, according to the present invention, comprising a hollow curved connector 1, a mask body or shell 21 defining an inner chamber 30 comprising a gas inlet 22 in fluid communication with said inner chamber 30 (cf. Fig. 2 & 3), and a front piece 24, commonly called "retainer" or "shroud", that is "sandwiched" between the tubular gas connector 1 and the mask body 21 of the mask 20.

The front shroud 24 comprises two lateral arms 26 and a holding arm 28, also called upper arm, that are integrally fixed to or molded in one-piece with the rest of the front shroud 24.

When the front shroud 24 is fixed to the mask body 21, the holding or upper arm 28 projects upwardly, i.e. about vertically, with respect to the mask body 21, whereas the two lateral arms 26 projecting laterally from said mask body 21 in opposite directions, i.e. one toward the right side of the mask 20 and the other toward the left side of the mask 20, as shown in Figures 1 to 3.

The two lateral arms 26 and the holding arm 28 have free distal ends carrying headgear connecting structures 25, such as slots, hooks or the like, so as to fix a headgear thereto. A headgear typically comprises several straps of fabric and/or polymer materials, or the like, and is used for securing and maintaining the mask 20 in position on the patient's face, when the mask is used, i.e. worn by the patient.

The holding arm 28 and/or of the two lateral arms 26 have typically elongated shapes, for example band or ribbon shapes, and are preferably made of a flexible material, such as a polymer material, e.g. polycarbonate (PC), polypropylene (PP) or nylon so as to be slightly bendable toward the patient's face under the forces applied by the headgear, when said headgear is adjusted to the morphology of the patient's head. The front shroud 24 can be made of the same polymer material or of any other suitable material.

As visible on Figures 2 and 3, the shroud 24 further comprises a central aperture 31 that is traversed by part of the hollow connector 1.

Furthermore, the mask body 21 can be made from a unique piece or from several pieces or sub-units that are subsequently assembled together and made integral by means of any available fixing technique, for instance they can be glued, thermo-welded or similarly affixed together. They can be made of a polymer material that is slightly flexible, preferably a polymer or plastic material, such as polypropylene (PP), polycarbonate (PC) and nylon, or similar.

Further, the facial mask 20 of the present invention also comprises a flexible cushion 23 that is fixed to the rear side of the mask body 21. Typically, the flexible cushion 23 is a tridimensional hollow structure forming a respiratory chamber 27 (cf. Fig. 3) with a front aperture 33 adapted, i.e. conformed and sized, for receiving at least part of the nose and mouth of the patient as above explained, when the patient wears the mask, so that the patient can breathe the respiratory gas contained in the inner chamber 30 of the mask body 21 and/or in the respiratory chamber 27 of the cushion 23 as those chambers 30, 27 are in fluid communication with one another.

Indeed, the cushion 23 comprises a cushion body 34 delimiting the respiratory chamber 27 and further comprises a rear aperture or rear opening 42 delimited by a rear border 41 (cf. Fig. 3), that opens into the inner chamber 30 of the mask body 21, when the cushion 23 is firmly fixed to the mask body 21, so that gas can freely pass from the inner chamber 30 of the mask body 21 towards the respiratory chamber 27 of the cushion 23, and vice versa. Actually, the inner chamber 30 of the mask body 21 and the respiratory chamber 27 of the cushion 23 form together a large compartment wherein the patient can inspire fresh gas, such as air, during inhalation phases, and exhale CO₂-enriched gas, during expiration phases.

In other words, the respiratory chamber 27 of the cushion 23 and the inner chamber 30 of the mask body 21 are both in fluid communication with the central gas passage 3, i.e. the lumen, of the elbow connector 1 of the invention so that a gas flow can freely travel from said tubular connector 1 to said chambers 27, 30, or vice versa.

In order to provide efficient gas tightness (i.e. seal) and/or increased comfort for the patient, the aperture of the cushion 23 is delimited by a flexible membrane 32 that comes into contact with the patient's face and spouses his/her facial morphology, when the mask 20 is worn by the patient. This membrane 32 constitutes a kind of soft flexible skirt delimiting the front aperture 33 of the cushion 23. Of course, depending on the embodiment, two or more superimposed membranes 32 can also be used as using several membranes may improve the gas tightness in certain circumstances, e.g. for some particular patient's morphologies.

The cushion 23 has, in the present case, a generally tridimensional (3D) trapezoidal shape, but other shapes are also possible such as triangular or saddle shapes, so as to better fit with the contours of the patient's face, especially in the nasal and mouth regions. When the facial mask 20 is worn by the patient, i.e. when the front aperture 33 of cushion 23 receives at least a part of the nose and the mouth of the patient, i.e., when the patient introduces his/her nose into the internal volume defined by the inner and the respiratory chambers 27, 30, and breathes the gas contained therein or exhales CO₂-enriched gases into it, the cushion 23 and the membrane 32 forming the peripheral border or skirt 35 of the front aperture 33 of the cushion 23 are in contact with particular and well defined regions of the patient's face, thereby ensuring gas tightness.

Preferably, the cushion 23 is a facial cushion configured and sized for coming into contact, when the mask is worn by a user, with at least the nasal bridge region (including lateral nose regions), the chin region, i.e. the area located under the lower lip, and the cheek regions located on both lateral sizes of the nose and mouth of the patient, said cheek regions linking the nasal bridge region including lateral nose regions to the chin region. Actually, the nasal bridge region can be an area of the nose located either at the junction of bone and cartilage, or below or above said bone/cartilage junction of the nose; the chin region is expending from the lower lip to the chin, whereas the cheek regions are the opposite areas located on right and left sizes of the nose and mouth. Of course, other embodiments and shapes of the cushion 23 are possible.

The cushion 23 (i.e., including cushion body 34 and membrane 32) is preferably made of a flexible and soft material, such as silicone or the like. Preferably, the membrane 32 and the cushion body 34 are molded in one piece.

As shown in Figure 3, the cushion 23 is fixed to the mask body 21 by means of a male/female connecting system, such as male/female connections, for instance peripheral groove(s) and wall(s) arranged on the rear border 41 of the cushion 23 cooperating with complementary peripheral wall(s) and/or groove(s) arranged on the rear border 40 of the mask body 21 of the mask 20 that are configured or shaped so as to fit together for ensuring for instance a snap-fit connection, a "sandwich-type connection or the like. They can also be glued together or the like.

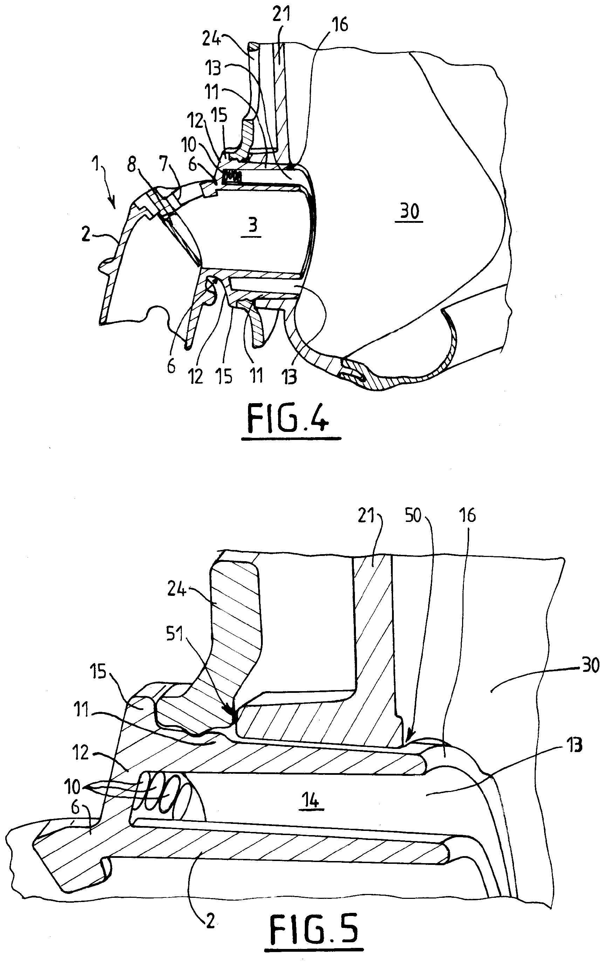

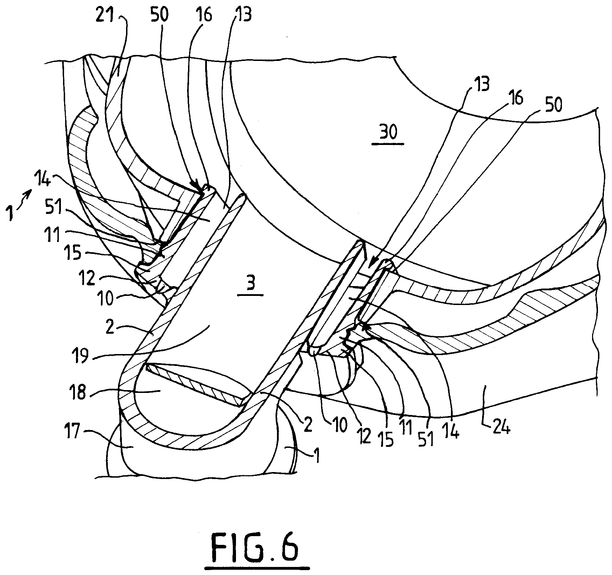

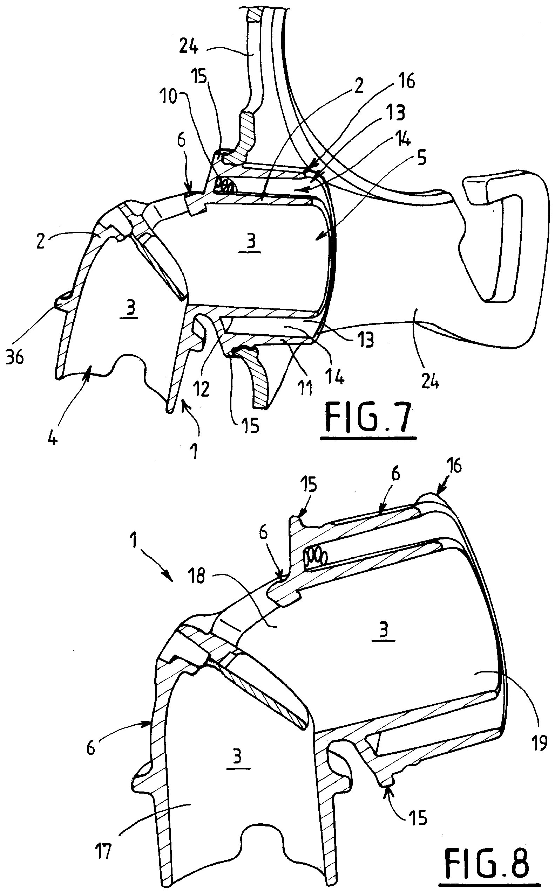

The gas inlet 22 of the mask body 21 is designed for fixing thereto a tubular gas connector 1 as shown in Fig. 4-8, so that the front shroud 24 is "sandwiched" and/or "squeezed" between the tubular connector 1 and the mask body 21.

The hollow connector 1 has a tubular body 2 traversed by an inner passage 3 comprising an inlet orifice 4 and an outlet orifice 5. The tubular body 2 is preferably curved so as to have a L- or elbow-shape. In other words, tubular body 2 comprises an upstream linear (i.e. straight) portion 17 comprising the inlet orifice 4, a downstream linear (i.e. straight) portion 19 comprising the outlet orifice 5 and an intermediary curved portion 18 comprising an elbow or the like, said intermediary curved portion 18 being located between the two linear portions 17, 19. The inner passage 3 passes through the two linear portions 17, 19 and the intermediary curved portion 18 thereby linking the inlet orifice 4 and the outlet orifice 5 so as to be able to convey fresh gas from the inlet orifice 4 to the outlet orifice 5. The inner passage 3 has generally a circular cross section.

In the case of a facial mask, it is wise that the connector 1 further comprises an anti-asphyxia system 7, 8 comprising a venting port 7 cooperating with a mobile flap 8, i.e. a flexible flap, so as to allow the patient breathing fresh air through the venting port 7 in case of failure of the gas source, such as a medical ventilator, as shown in Fig. 1-3. Such asphyxia system 7, 8 are known in the art, as disclosed by US5438981 or WO-A-00/38772.

The upstream portion of the tubular body 2 of the hollow connector 1, that comprises the inlet orifice 4, is fed with gas, such as pressurized air, by a flexible hose (not shown) or the like conveying the gas delivered by a medical gas source, such as a medical ventilator or apparatus. The flexible hose is fluidly connected to the tubular body 2 of the hollow connector 1 of the invention by means of an intermediary connector 29, i.e. a tubing element, that is connected, on the one hand, to the upstream portion of the tubular body 2 of the hollow connector 1, for instance connected by a snap-fit system, and, on the other hand, to the flexible hose conveying the respiratory gas. For limiting the course of the intermediary connector 29, while it is connected, i.e. snap-fitted or similar, to the upstream portion of the tubular body 2 of the hollow connector 1, a annular abutment 36 is provided around the upstream portion of the tubular body 2, which forms an annular border or ring, as shown in Fig. 2, 3 or 7. The intermediary connector 29 has generally tubular shape and is made of a polymer material. It is further coaxially arranged to the upstream portion of the tubular body 2 of the hollow connector 1 and is preferably rotatable with respect to the hollow curved connector 1 according to the present invention.

In the embodiment shown in Fig. 4-8, the tubular body 2 of the curved connector 1 further comprises a tubular sleeve 11, i.e. a tube (or pipe) element or the like, that is arranged around the tubular body 2, namely around the downstream linear portion comprising the outlet orifice 5. Preferably, the tube element 11 is coaxially arranged around the tubular body 2 of the hollow connector 1. The tubular sleeve 11 comprises a free open end 13 and a blind end 12, and delimits a venting passage 14 between the tubular sleeve 11 and the tubular body 2. The free open end 13 of the tubular sleeve 11 is arranged around the outlet orifice 5 of the tubular body 2, i.e. the free open end 13 of the tubular sleeve 11 and the outlet orifice 5 of the tubular body 2 are coaxially arranged.

The tubular sleeve 11 further comprises a blind end 12 that is located opposite to the free end 13 and that is fixed to the peripheral outer wall 6 of the tubular body 2, thereby forming a bottom or closing wall of the tubular sleeve 11.

The tubular sleeve 11 is arranged at a distance of less than 10 mm, typically of about 5 mm or less, from the peripheral outer wall 6, i.e. the outer surface, of the tubular body 2 so as to create or delimit between them a venting passage 14 for recovering CO₂-riched expired gases exhaled by the patient. The venting passage 14 has hence an annular cross-section. The exhaled gas enters into the venting passage 14 by the free open end 13 of the tubular sleeve 11, while the patient exhales, and travels therein toward the venting orifices 10 through which the expired CO₂-rich gas is vented to the atmosphere.

A plurality of venting holes 10 are arranged in the blind end 12 of the tubular sleeve 11. Those venting holes 10 fluidly communicate, on the one hand, with the venting passage 14 delimited by the inner surface of the tubular sleeve 11 and the outer surface, i.e. the peripheral outer wall 6, of the tubular body 2, and, on the other hand, with the ambient atmosphere, i.e. ambient air, surrounding the connector 1. In other words, the plurality of venting holes 10 puts the venting passage 14 in fluid communication with the ambient atmosphere so as to wash out expired-gas present into the venting passage 14. Preferably, the venting holes 10 have a (tron)conical shape, i.e. an enlarged section (i.e., diameter) on their inlet side than on their outlet side so as to facilitate the gas venting.

The venting holes 10 can be arranged in an array forming a circle or preferably an arc of circle (i.e., semi-circular array) in the blind end 12 of the tubular sleeve 11 as shown in Fig. 1. In other words, the venting ports 10 are distributed all along the circumference of the closed bottom or blind end 12 of the tubular sleeve 11 or preferably along only a part of that circumference as represented in Fig. 1-3. Of course, holes 10 can also be arranged in several arrays, e.g. coaxially arranged arrays, or in any other way. Preferably, at least 5 venting holes are provided, more preferably at least 10 venting holes 10, advantageously between 12 and 30 venting holes 10.

The different elements of the connector 1, especially the tubular sleeve 11 and the tubular body 2 are preferably molded in one piece ; however, they can also be made of several elements or pieces, i.e. sub-units, that are fixed together, e.g., glued or the like. Preferably, the connector 1 including the tubular sleeve 11 and the tubular body 2 is entirely made of a plastic material, such as PP, PC or nylon.

In the embodiment of Fig. 4-8, the tubular sleeve 11 is attached to that annular shoulder 15 so that the tubular sleeve 11 constitutes also the blind end 12 of the tubular sleeve 11, i.e. the closed bottom 12 of the tubular sleeve 11. The venting holes 10 are hence arranged (e.g. bored) through said annular shoulder 15.

More generally speaking, the hollow curved connector 1 comprises an upstream portion 17 and a downstream portion 19 that are usually almost linear, i.e. straight, and that separated by an elbow portion 18, i.e. bent portion. The hollow curved connector 1 is axially traversed by an inner passage 3 having an inlet orifice 4 located at the free end of the upstream portion 17, and an outlet orifice 5 located at the free end of the downstream portion19.

The downstream portion 19 is fluidly connected to the mask body 21 for feeding gas into the inner chamber 30. In other words, the gas that passes through the inner passage 3 of the hollow curved connector 1 can be delivered into the inner chamber 30 of the mask body 21 for being inhaled by a patient wearing the mask 20.

The front piece or shroud 24 comprises a central aperture 31 or opening and several headgear connecting structures 25, such as hooks and/or slots or similar, for receiving the straps of a headgear (not shown).

According to the present invention, the gas inlet 22 of the mask body 21 and the central aperture 31 of the front piece or shroud 24 are traversed by the downstream portion 19 of the hollow connector 1 as shown in Fig. 4-6.

It is further provided a first abutment structure 15 and a second abutment structure 16 that are arranged on the peripheral outer wall 6, i.e. the external surface, of the downstream portion 19 of the hollow connector 1. Those abutments structures 15, 16 allow holding the front shroud 24 integral with the mask body 21 and the curved connector 1.

In this goal, the second abutment structure 16 cooperates with the inner wall 50 of the mask body 21, in a region situated around the gas inlet 22, i.e. around the gas inlet 22, for retaining/holding the hollow connector 1 integral with the mask body 21, while squeezing the front piece 24 between the mask body 21 and the first abutment structure 15, when the downstream portion 19 of the hollow connector 1 is inserted into the gas inlet 22 of the mask body 21.

Actually, the front piece 24 is sandwiched between a collar element 52 arranged around the gas inlet 22 of the mask body 21 and the first abutment structure 15. The collar element 52 is part of the external wall, i.e. external surface, of the mask body 21.

In other words, the front piece 24 is hold tight in position as being squeezed between the first abutment structure 15 and the mask body 21, namely between the first abutment structure 15 and the collar element 52 arranged around the gas inlet 22 of the mask body 21, whereas the assembly comprising the front piece 24, the hollow curved connector 1 and the mask body 21 are hold tight together thanks to the cooperation, i.e. grip, of the second abutment structure 16 with the inner surface or wall, of the mask body 21 in the region located around the gas inlet 22 inside the mask body 21.

Such an arrangement according to the present invention does not require any positive connection of the front piece 24 with the mask body 21, i.e. no snap-fit connection or the like. Indeed, the holding of those elements is ensured by the hollow curved connector 1 that, once inserted through the aperture of the front piece 31 and the gas inlet 22 of the mask body 21, firmly maintains those elements in place, i.e. "sandwiching" the front piece 24 between the collar element 52 of the mask body and the first abutment structure 15 arranged on the outer surface of the hollow curved connector 1, whereas the hollow curved connector is itself hold in position into the gas inlet 22 by the second abutment structure 16, also arranged on its outer surface 6, that abuts against the internal wall 50, i.e. the inner surface, of the mask body 21 in the region located around the gas inlet 22 inside the mask body 21.

Preferably, the first abutment structure 15 and the second abutment structure 16 can comprise a ring structure or at least a portion (i.e. a part) of a ring structure arranged around the peripheral surface of the downstream portion 18.

Further, the downstream portion 19 of the hollow connector 1 can be snap-fitted into the gas inlet 22 of the mask body 21 so that the second abutment structure 16 can abut against the internal wall 50, i.e. the inner surface, of the mask body 21 and prohibit any undesired withdrawal of the hollow connector 1.

Of course, the first abutment structure 15 is sized so as to exhibit a first gripping surface that is sufficient for abutting against the front shroud 24 and for holding it firmly, whereas the second abutment structure 16 is sized so as to present a second gripping surface that is sufficient for abutting against the inner wall of the mask body 21, in the area located around the inlet 22, and thereby holding the hollow curved connector 1 integral with the mask body 21, while sandwiching the front shroud 24 between the mask body 21, namely the collar element 52, i.e. a cylindrical tube piece, surrounding the inlet 22 of the mask body 21, and the first abutment structure 15 carried by the connector 1.

Depending on the structure or embodiment of the hollow curved connector 1, the first abutment structure 15 and a second abutment structure 16 can project away either from the peripheral outer wall 6 of the downstream portion 19 of the hollow connector 1, or from on the outer surface 6 of the tubular sleeve 11 surrounding the tubular body 2 of the hollow connector 1.

Generally speaking, the respiratory mask 20 of the present invention, such as a nasal or facial mask, is light, comfortable to wear, easy to position and secure on the patient's face, provide a good tightness (seal) and an efficient CO₂-enriched gas venting to the atmosphere.

The respiratory mask of the present invention can be used in a method for treatment of a respiratory disorder or condition affecting infant, toddler and child patients as well as adult patients, for example in non-invasive positive pressure ventilation (NPPV) or in a nasal continuous positive airway pressure (N-CPAP) therapy of sleep disordered breathing (SDB) conditions, such as, for example, obstructive sleep apnea (OSA).

## Claims

1. Respiratory mask (20) comprising:
- a mask body (21) comprising an inner chamber (30) and a gas inlet (22) in fluid communication with said inner chamber (30),
- a hollow curved connector (1) comprising an upstream portion (17) and a downstream portion (19) separated by an elbow portion (18), said hollow curved connector (1) being traversed by an inner passage (3) comprising an inlet orifice (4) in the upstream portion (17) and an outlet orifice (5) in the downstream portion (19), said downstream portion (19) being fluidly connected to the mask body (21) for feeding gas into the inner chamber (30), and
- a front piece (24) comprising a central aperture (31) and headgear connecting structures (25),
and wherein the gas inlet (22) of the mask body (21) and the central aperture (31) of the front piece (24) are traversed by the downstream portion (19) of the hollow connector (1), and the peripheral outer wall (6) of the downstream portion (19) of the hollow connector (1) is shaped so as to present a first abutment structure (15) and a second abutment structure (16), said first and second abutment structures (15, 16) cooperating with the front piece (24) and the mask body (21) for holding the hollow connector (1) and the front piece (24) integral with the mask body (21), when the downstream portion (19) of the hollow connector (1) is inserted into the gas inlet (22) of the mask body (21),
**characterized in that** the front piece (24) is hold tight in position as being squeezed between the first abutment structure (15) and the mask body (2), and the assembly comprising the front piece (24), the hollow curved connector (1) and the mask body (21) is further hold tight together thanks to the cooperation of the second abutment structure (16) with the inner wall (50) of the mask body (21) in the region located around the gas inlet (22) inside the mask body (21), without requiring any positive connection of the front piece (24) with the mask body (21).

2. Respiratory mask (20) according to the preceding claim, **characterized in that** the second abutment structure (16) cooperates with the inner wall (50) of the mask body (21) in a region situated around the gas inlet (22), for retaining the hollow connector (1) and the front piece (24) integral with the mask body (21), and for squeezing the front piece (24) between the mask body (21) and the first abutment structure (15).

3. Respiratory mask (20) according to anyone of the preceding claims, **characterized in that** the outer wall (51) of the mask body (21) comprises a collar element (52) arranged around the gas inlet (22) of the mask body (21), the front piece (24) being squeezed against the first abutment structure (15) by said collar element (52).

4. Respiratory mask (20) according to anyone of the preceding claims, **characterized in that** the first abutment structure (15) and the second abutment structure (16) project away from the peripheral outer wall (6) of the downstream portion (19).

5. Respiratory mask (20) according to anyone of the preceding claims, **characterized in that** the first abutment structure (15) and the second abutment structure (16) are arranged at a distance from each other of at least 4 mm.

6. Respiratory mask (20) according to anyone of the preceding claims, **characterized in that** the first abutment structure (15) and/or the second abutment structure (16) comprise a ring structure or at least a portion of a ring structure arranged around the peripheral surface of the downstream portion (18).

7. Respiratory mask (20) according to anyone of the preceding claims, **characterized in that** the downstream portion (19) of the hollow connector (1) is snap-fitted into the gas inlet (22) of the mask body (21).

8. Respiratory mask (20) according to anyone of the preceding claims, **characterized in that** the downstream portion (19) of the hollow connector (1) comprises a tubular body (2) traversed by the inner passage (3) and a tubular sleeve (11) is arranged around the tubular body (2), said tubular sleeve (11) comprising a free open end (13) and a blind end (12), and delimits a venting passage (14) between the tubular sleeve (11) and the tubular body (2), the free open end (13) of the tubular sleeve (11) being arranged around the outlet orifice (5) of the tubular body (2), said tubular sleeve (11) being fixed, by the blind end (12), to the peripheral outer wall (6) of the tubular body (2), the first abutment structure (15) comprising said blind end (12) of the tubular sleeve (11), a plurality of venting holes (10) putting the venting passage (14) in fluid communication with the atmosphere being arranged in said blind end (12).

9. Respiratory mask (20) according to anyone of the preceding claims, **characterized in that** the first abutment structure (15) and a second abutment structure (16) project away from the peripheral outer wall (6) of the downstream portion (19) of the hollow connector (1).

10. Respiratory mask (20) according to anyone of the preceding claims, **characterized in that** the second abutment structure (16) is arranged on the outer surface of the tubular sleeve (11) surrounding the tubular body (2) of the hollow connector (1).

11. Respiratory mask (20) according to anyone of the claims 1 to 9, **characterized in that** the second abutment structure (16) is arranged on the outer surface of the tubular body (2) of the hollow connector (1).

12. Respiratory mask (20) according to anyone of the preceding claims, **characterized in that** the hollow connector (1) further comprises an anti-asphyxia system (7, 8) comprising a venting port (7) cooperating with a mobile flap (8).

13. Respiratory mask (20) according to anyone of the preceding claims, **characterized in that** it further comprises :
- a cushion (23) fixed to the mask body (21), and/or
- a headgear fixed to the headgear connecting structures (25) of the front element (24).

14. Respiratory mask (20) according to anyone of the preceding claims, **characterized in that** it is a facial mask.

15. Assembly for delivering a gas to a patient comprising a gas delivery device, such as a medical ventilator, and a respiratory mask according to anyone of the preceding claims, the gas delivery device being fluidly connected to the respiratory mask by means of a gas line, such as a flexible hose.

## Patentansprüche

1. Atemmaske (20), umfassend:
- einen Maskenkörper (21), umfassend eine Innenkammer (30) und einen Gaseinlass (22) in Fluidkommunikation mit der Innenkammer (30),
- einen hohlen gekrümmten Stutzen (1), umfassend einen stromaufwärtigen Abschnitt (17) und einen stromabwärtigen Abschnitt (19), getrennt durch einen Ellbogenabschnitt (18), wobei der hohle gekrümmte Stutzen (1) von einem Innendurchgang (3) durchquert wird, umfassend eine Einlassöffnung (4) in dem stromaufwärtigen Abschnitt (17) und eine Auslassöffnung (5) in dem stromabwärtigen Abschnitt (19), wobei der stromabwärtige Abschnitt (19) zum Zuführen von Gas in die Innenkammer (30) fluidisch mit dem Maskenkörper (21) verbunden ist, und
- ein vorderseitiges Teil (24), umfassend eine zentrale Apertur (31) und Kopfbedeckung-Verbindungsstrukturen (25),
und wobei der Gaseinlass (22) des Maskenkörpers (21) und die zentrale Apertur (31) des vorderseitigen Teils (24) von dem stromabwärtigen Abschnitt (19) des hohlen Stutzens (1) durchquert werden und die umfängliche Außenwand (6) des stromabwärtigen Abschnitts (19) des hohlen Stutzens (1) geformt ist, um eine erste Anschlagsstruktur (15) und eine zweite Anschlagsstruktur (16) aufzuweisen, wobei die erste und zweite Anschlagsstruktur (15, 16) mit dem vorderseitigen Teil (24) und dem Maskenkörper (21) zusammenwirken, um den hohlen Stutzen (1) und das vorderseitige Teil (24) einstückig mit dem Maskenkörper (21) zu halten, wenn der stromabwärtige Abschnitt (19) des hohlen Stutzens (1) in den Gaseinlass (22) des Maskenkörpers (21) eingesetzt wird,
**dadurch gekennzeichnet, dass** das vorderseitige Teil (24) fest in Position gehalten wird, wie zwischen die erste Anschlagsstruktur (15) und den Maskenkörper (2) gedrückt wird, und die Anordnung das vorderseitige Teil (24) umfasst, wobei der hohle gekrümmte Stutzen (1) und der Maskenkörper (21) weiter Dank dem Zusammenwirken der zweiten Anschlagsstruktur (16) mit der Innenwand (50) des Maskenkörpers (21) in dem Bereich, der sich um den Gaseinlass (22) herum innerhalb des Maskenkörper (21) befindet, ohne jegliche positive Verbindung des vorderseitigen Teils (24) mit dem Maskenkörper (21), fest zusammengehalten wird.

2. Atemmaske (20) nach dem vorstehenden Anspruch, **dadurch gekennzeichnet, dass** die zweite Anschlagsstruktur (16) mit der Innenwand (50) des Maskenkörpers (21) in einem Bereich, der um den Gaseinlass (22) herum situiert ist, zusammenwirkt, um den hohlen Stutzen (1) und das vorderseitige Teil (24) mit dem Maskenkörper (21) einstückig beizubehalten, und zum Drücken des vorderseitigen Teils (24) zwischen den Maskenkörper (21) und die erste Anschlagsstruktur (15).

3. Atemmaske (20) nach einem der vorstehenden Ansprüche, **dadurch gekennzeichnet, dass** die Außenwand (51) des Maskenkörpers (21) ein um den Gaseinlass (22) des Maskenkörpers (21) herum angeordnetes Kragenelement (52) umfasst, wobei das vorderseitige Teil (24) durch das Kragenelement (52) gegen die erste Anschlagsstruktur (15) gedrückt wird.

4. Atemmaske (20) nach einem der vorstehenden Ansprüche, **dadurch gekennzeichnet, dass** die erste Anschlagsstruktur (15) und die zweite Anschlagsstruktur (16) von der umfänglichen Außenwand (6) des stromabwärtigen Abschnitts (19) weg abstehen.

5. Atemmaske (20) nach einem der vorstehenden Ansprüche, **dadurch gekennzeichnet, dass** die erste Anschlagsstruktur (15) und die zweite Anschlagsstruktur (16) in einem Abstand von mindestens 4 mm voneinander angeordnet sind.

6. Atemmaske (20) nach einem der vorstehenden Ansprüche, **dadurch gekennzeichnet, dass** die erste Anschlagsstruktur (15) und/oder die zweite Anschlagsstruktur (16) eine Ringstruktur oder mindestens einen Abschnitt einer Ringstruktur, um die umfängliche Oberfläche des stromabwärtigen Abschnitts (18) herum angeordnet, umfassen.

7. Atemmaske (20) nach einem der vorstehenden Ansprüche, **dadurch gekennzeichnet, dass** der stromabwärtige Abschnitt (19) des hohlen Stutzens (1) in den Gaseinlass (22) des Maskenkörpers (21) eingerastet ist.

8. Atemmaske (20) nach einem der vorstehenden Ansprüche, **dadurch gekennzeichnet, dass** der stromabwärtige Abschnitt (19) des hohlen Stutzens (1) einen durch den Innendurchgang (3) durchquerten röhrenförmigen Körper (2) umfasst und eine röhrenförmige Hülse (11) um den röhrenförmigen Körper (2) herum angeordnet ist, wobei die röhrenförmige Hülse (11) ein freies offenes Ende (13) und ein blindes Ende (12) umfasst, und einen Belüftungsdurchgang (14) zwischen der röhrenförmigen Hülse (11) und dem röhrenförmigen Körper (2) eingrenzt, wobei das freie offene Ende (13) der röhrenförmigen Hülse (11) um die Auslassöffnung (5) des röhrenförmigen Körpers (2) angeordnet ist, wobei die röhrenförmige Hülse (11) durch das blinde Ende (12) an der umfänglichen Außenwand (6) des röhrenförmigen Körpers (2) fixiert ist, wobei die erste Anschlagsstruktur (15) das blinde Ende (12) der röhrenförmigen Hülse (11) umfasst, wobei eine Vielzahl von Belüftungslöchern (10), die den Belüftungsdurchgang (14) in Fluidkommunikation mit der Atmosphäre bringen, in dem blinden Ende (12) angeordnet ist.

9. Atemmaske (20) nach einem der vorstehenden Ansprüche, **dadurch gekennzeichnet, dass** die erste Anschlagsstruktur (15) und eine zweite Anschlagsstruktur (16) von der umfänglichen Außenwand (6) des stromabwärtigen Abschnitts (19) des hohlen Stutzens (1) weg abstehen.

10. Atemmaske (20) nach einem der vorstehenden Ansprüche, **dadurch gekennzeichnet, dass** die zweite Anschlagsstruktur (16) auf der Außenoberfläche der röhrenförmigen Hülse (11), die den röhrenförmigen Körper (2) des hohlen Stutzens (1) umgibt, angeordnet ist.

11. Atemmaske (20) nach einem der Ansprüche 1 bis 9, **dadurch gekennzeichnet, dass** die zweite Anschlagsstruktur (16) auf der Außenoberfläche des röhrenförmigen Körpers (2) des hohlen Stutzens (1) angeordnet ist.

12. Atemmaske (20) nach einem der vorstehenden Ansprüche, **dadurch gekennzeichnet, dass** der hohle Stutzen (1) weiter ein Anti-Erstickungssystem (7, 8) umfasst, umfassend einen Belüftungsanschluss (7), der mit einer mobilen Klappe (8) zusammenwirkt.

13. Atemmaske (20) nach einem der vorstehenden Ansprüche, **dadurch gekennzeichnet, dass** sie weiter umfasst:
- ein an den Maskenkörper (21) fixiertes Polster (23) und/oder
- eine an die Kopfbedeckung-Verbindungsstrukturen (25) des vorderseitigen Elements (24) fixierte Kopfbedeckung.

14. Atemmaske (20) nach einem der vorstehenden Ansprüche, **dadurch gekennzeichnet, dass** sie eine Gesichtsmaske ist.

15. Anordnung zum Abgeben eines Gases an einen Patienten, umfassend eine Gasabgabevorrichtung, wie ein medizinisches Beatmungsgerät, und eine Atemmaske nach einem der vorstehenden Ansprüche, wobei die Gasabgabevorrichtung mittels einer Gasleitung, wie einem flexiblen Schlauch, fluidisch mit der Atemmaske verbunden ist.

## Revendications

1. Masque respiratoire (20) comprenant :
- un corps de masque (21) comprenant une chambre intérieure (30) et une entrée de gaz (22) en communication fluidique avec ladite chambre intérieure (30),
- un raccord incurvé creux (1) comprenant une portion amont (17) et une portion aval (19) séparées par une portion coudée (18), ledit raccord incurvé creux (1) étant traversé par un passage interne (3) comprenant un orifice d'entrée (4) dans la portion amont (17) et un orifice de sortie (5) dans la portion aval (19), ladite portion aval (19) étant reliée de manière fluidique au corps de masque (21) pour introduire du gaz dans la chambre intérieure (30), et
- un élément frontal (24) comprenant une ouverture centrale (31) et des structures de liaison de harnais (25),
et dans lequel l'entrée de gaz (22) du corps de masque (21) et l'ouverture centrale (31) de l'élément frontal (24) sont traversées par la portion aval (19) du raccord creux (1), et la paroi extérieure périphérique (6) de la portion aval (19) du raccord creux (1) est formée de manière à présenter une première structure de butée (15) et une seconde structure de butée (16), lesdites première et seconde structures de butée (15, 16) coopérant avec l'élément frontal (24) et le corps de masque (21) pour maintenir le raccord creux (1) et l'élément frontal (24) d'un seul tenant avec le corps de masque (21), lorsque la portion aval (19) du raccord creux (1) est insérée dans l'entrée de gaz (22) du corps de masque (21),
**caractérisé en ce que** l'élément frontal (24) est maintenu fermement en position sous l'effet d'une compression entre la première structure de butée (15) et le corps de masque (2), et l'ensemble comprenant l'élément frontal (24), le raccord incurvé creux (1) et le corps de masque (21) sont maintenus en outre fermement ensemble grâce à la coopération de la seconde structure de butée (16) avec la paroi intérieure (50) du corps de masque (21) dans la zone située autour de l'entrée de gaz (22) à l'intérieur du corps de masque (21), sans nécessiter une quelconque liaison par forme de l'élément frontal (24) avec le corps de masque (21).

2. Masque respiratoire (20) selon la revendication précédente, **caractérisé en ce que** la seconde structure de butée (16) coopère avec la paroi intérieure (50) du corps de masque (21) dans une zone située autour de l'entrée de gaz (22), permettant de maintenir le raccord creux (1) et l'élément frontal (24) d'un seul tenant avec le corps de masque (21), et permettant de comprimer l'élément frontal (24) entre le corps de masque (21) et la première structure de butée (15).

3. Masque respiratoire (20) selon l'une quelconque des revendications précédentes, **caractérisé en ce que** la paroi extérieure (51) du corps de masque (21) comprend un élément formant collier (52) agencé atour de l'entrée de gaz (22) du corps de masque (21), l'élément frontal (24) étant comprimé contre la première structure de butée (15) par ledit élément formant collier (52).

4. Masque respiratoire (20) selon l'une quelconque des revendications précédentes, **caractérisé en ce que** la première structure de butée (15) et la seconde structure de butée (16) se projettent à distance de la paroi extérieure périphérique (6) de la portion aval (19).

5. Masque respiratoire (20) selon l'une quelconque des revendications précédentes, **caractérisé en ce que** la première structure de butée (15) et la seconde structure de butée (16) sont agencées à une distance l'une de l'autre d'au moins 4 mm.

6. Masque respiratoire (20) selon l'une quelconque des revendications précédentes, **caractérisé en ce que** la première structure de butée (15) et/ou la seconde structure de butée (16) comprennent/comprend une structure annulaire ou au moins une portion d'une structure annulaire agencée autour de la surface périphérique de la portion aval (18).

7. Masque respiratoire (20) selon l'une quelconque des revendications précédentes, **caractérisé en ce que** la portion aval (19) du raccord creux (1) est adaptée par encliquetage dans l'entrée de gaz (22) du corps de masque (21).

8. Masque respiratoire (20) selon l'une quelconque des revendications précédentes, **caractérisé en ce que** la portion aval (19) du raccord creux (1) comprend un corps tubulaire (2) traversé par le passage interne (3) et un manchon tubulaire (11) est agencé autour du corps tubulaire (2), ledit manchon tubulaire (11) comprenant une extrémité ouverte libre (13) et une extrémité fermée (12), et délimite un passage de ventilation (14) entre le manchon tubulaire (11) et le corps tubulaire (2), l'extrémité ouverte libre (13) du manchon tubulaire (11) étant agencée autour de l'orifice de sortie (5) du corps tubulaire (2), ledit manchon tubulaire (11) étant fixé, par l'extrémité fermée (12), à la paroi extérieure périphérique (6) du corps tubulaire (2), la première structure de butée (15) comprenant ladite extrémité fermée (12) du manchon tubulaire (11), une pluralité de trous de ventilation (10) mettant le passage de ventilation (14) en communication fluidique avec l'atmosphère étant agencés dans ladite extrémité fermée (12).

9. Masque respiratoire (20) selon l'une quelconque des revendications précédentes, **caractérisé en ce que** la première structure de butée (15) et une seconde structure de butée (16) se projettent à distance de la paroi extérieure périphérique (6) de la portion aval (19) du raccord creux (1).

10. Masque respiratoire (20) selon l'une quelconque des revendications précédentes, **caractérisé en ce que** la seconde structure de butée (16) est agencée sur la surface extérieure du manchon tubulaire (11) entourant le corps tubulaire (2) du raccord creux (1).

11. Masque respiratoire (20) selon l'une quelconque des revendications 1 à 9, **caractérisé en ce que** la seconde structure de butée (16) est agencée sur la surface extérieure du corps tubulaire (2) du raccord creux (1).

12. Masque respiratoire (20) selon l'une quelconque des revendications précédentes, **caractérisé en ce que** le raccord creux (1) comprend en outre un système anti-asphyxie (7, 8) comprenant un port de ventilation (7) coopérant avec un volet mobile (8).

13. Masque respiratoire (20) selon l'une quelconque des revendications précédentes, **caractérisé en ce qu'**il comprend en outre :
- un coussinet (23) fixé au corps de masque (21) et/ou
- un harnais fixé aux structures de liaison de harnais (25) de l'élément frontal (24).

14. Masque respiratoire (20) selon l'une quelconque des revendications précédentes, **caractérisé en ce qu'**il est un masque facial.

15. Ensemble destiné à administrer un gaz à un patient comprenant un dispositif d'administration de gaz, tel qu'un respirateur artificiel, et un masque respiratoire selon l'une quelconque des revendications précédentes, le dispositif d'administration de gaz étant relié de manière fluidique au masque respiratoire au moyen d'une ligne de gaz, telle qu'un tuyau souple.
